# EUROPEAN PATENT SPECIFICATION

(11) **EP 3 126 326 B1**
(45) Date of publication and mention of the grant of the patent: **09.05.2018**
(21) Application number: 15716927.7
(22) Date of filing: 26.03.2015
(51) Int. Cl.: C07C 253/24, F28D 7/08

(54) **COOLING COIL DESIGN FOR OXIDATION OR AMMOXIDATION REACTORS**
ENTWURF EINER KÜHLSCHLANGE FÜR OXIDATIONS- ODER AMMOXIDATIONSREAKTOREN
CONCEPTION DE SERPENTIN DE REFROIDISSEMENT POUR RÉACTEURS D'OXYDATION OU D'AMMOXYDATION

(30) Priority: 31.03.2014 CN 201410125138
(43) Date of publication of application: 08.02.2017
(73) Proprietor: Ineos Europe AG, 1180 Rolle (Vaud) (CH)
(72) Inventor: MCDONEL, Timothy Robert, Elburn, Illinois 60119 (US); COUCH, Jay Robert, Naperville, Illinois 60564 (US); WAGNER, David Rudolph, Naperville, Illinois 60564 (US); WACHTENDORF, Paul Trigg, Victoria, Texas 77904 (US); TRAVERS, Thomas George, Carpentersville, Illinois 60110 (US)
(74) Representative: King, Alex
(86) International application number: PCT/US2015/022703
(87) International publication number: WO 2015/153275

(56) References cited:
- US-A- 5 520 891
- US-A1- 2009 163 756

## Description

### Background

Various processes and systems for the manufacture of acrylonitrile and methacrylonitrile are known. Conventional processes typically involve recovery and purification of acrylonitrile/methacrylonitrile produced by a direct reaction of a hydrocarbon selected from the group consisting of propane, propylene or isobutylene, ammonia and oxygen in the presence of a catalyst. For example, in the commercial manufacture of acrylonitrile, propylene, ammonia and oxygen are reacted together according to the following reaction scheme:

CH₂=CH-CH₃ + NH₃ + 3/2 O₂ → CH₂=CH-CN + 3 H₂O

This process, which is commonly referred to as ammoxidation, is carried out in the gas phase at elevated temperature (*e.g.,* 350° to 480° C) in the presence of a suitable fluid bed ammoxidation catalyst.

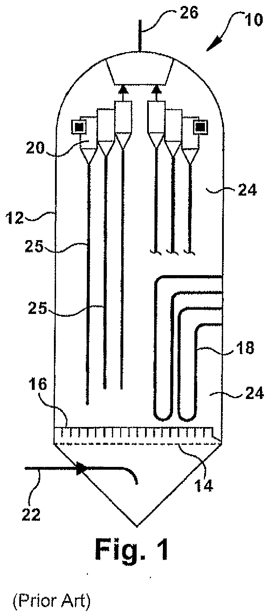

Fig. 1 illustrates a typical acrylonitrile reactor used to carry out this process. As shown there, reactor 10 comprises reactor shell 12, air grid 14, feed sparger 16, cooling coils 18 and cyclones 20. During normal operation, process air is charged into reactor 10 through air inlet 22, while a mixture of propylene and ammonia is charged into reactor 10 through feed sparger 16. The flow rates of both are high enough to fluidize a bed 24 of ammoxidation catalyst in the reactor interior, where the catalytic ammoxidation of the propylene and ammonia to acrylonitrile occurs.

Product gases produced by the reaction exit reactor 10 through reactor effluent outlet 26. Before doing so, they pass through cyclones 20, which remove any ammoxidation catalyst these gases may have entrained for return to catalyst bed 24 through diplegs 25. Ammoxidation is highly exothermic, and so a cooling coil assembly 18 is used to withdraw excess heat and thereby keep the reaction temperature at an appropriate level.

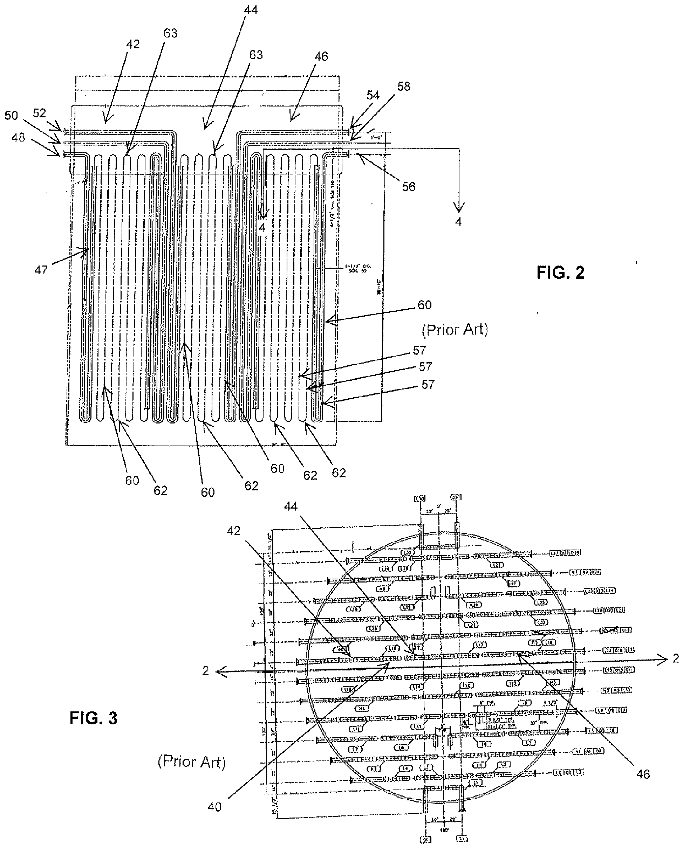

In this regard, Fig. 2 schematically illustrates the design of a conventional cooling coil assembly 18 which is used for this purpose. Figure 2 is a partial axial cross-sectional view of reactor 10, which illustrates one set of cooling coils in the cooling coil assembly of reactor 10, this set of cooling coils being composed of three separate cooling coils, cooling coil 42, cooling coil 44 and cooling coil 46. Cooling coil 42 includes an inlet 48 for receiving cooling water and an outlet 50 for discharging this cooling water after being heated and partially converted to steam. Similarly, cooling coil 44 includes inlet 52 and exit 54, while cooling coil 46 includes inlet 56 and outlet 58. As shown in Fig. 2, each of cooling coils 42, 44 and 46 is defined by a series of vertically-oriented cooling coil courses 57, each course being composed of a pair of elongated, interconnected cooling conduits 60 which are connected to one another at their bottoms by lower U-turn fitting 62. Successive coiling coil courses 57 are also connected to one another at their tops by upper U-turn fittings 63 so as to form a continuous flow passageway from the inlet to the outlet of each respective cooling coil.

Figure 3 is a top view of cooling coil assembly 18 illustrated in Fig. 2. As will be appreciated from Figs. 2 and 3, cooling coils 42, 44 and 46 form a set of cooling coils which are co-planar, *i.e.,* each lies in a common vertical plane. As further illustrated in Fig. 3, cooling coil assembly 18 is composed of multiple sets of these cooling coils, with each set of these cooling coils being arranged generally parallel to, and (optionally) equally spaced from, one another. Moreover, as can be further seen in Figure 3, while many of the cooling coil sets in this cooling coil assembly include three different cooling coils, other cooling coils sets include two or four cooling coils, while two cooling coil sets contain only one cooling coil.

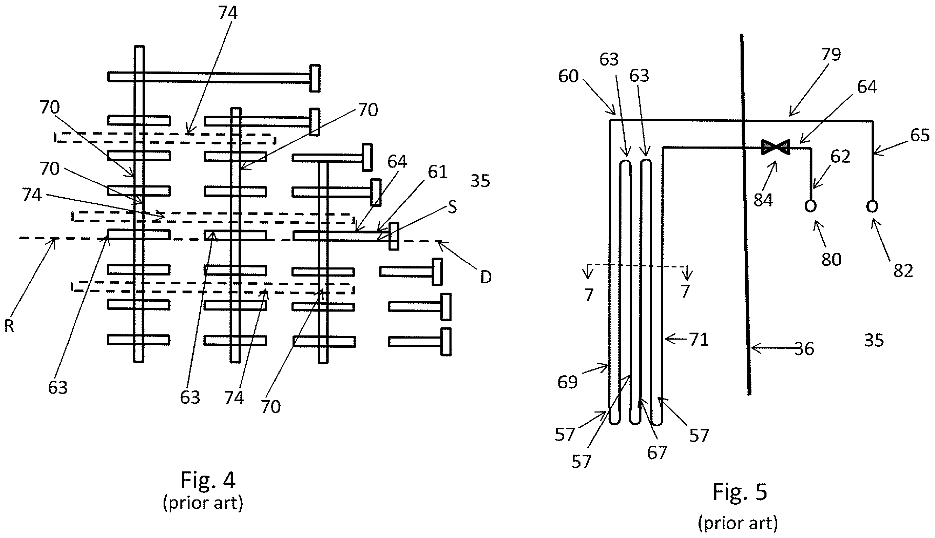

Fig. 4 is an enlarged top view taken on line 4-4 of Fig. 2, showing more detail of the particular structure of cooling coil assembly 18 of Figs 2 and 3. In particular, Fig 4 is a schematic view in which only upper U-turn fittings 63 of the cooling coils are shown.

As shown in Fig. 4, cooling coil 61 includes an inlet 35, a supply line 64 connecting inlet 35 to the top of the first cooling coil course (not shown) of cooling coil 61 and a series of upper U-turn fittings 63 for connecting the successive coil courses of the cooling coil to one another. As also shown in this figure, all of these elements, *i.e.,* all of the upper U-turn fittings 63 as well as supply line 64 are co planar, *i.e.,* they all lie in the same common vertical plane D. Moreover, from Figs. 2 and 3, it will be further appreciated that the remaining elements in this cooling coil, *i.e.,* the vertically oriented cooling conduits 60 forming each cooling coil course 57 and the associated lower U-turn fittings 62, also lie in this common vertical plane. In the particular embodiment shown, each upper U-turn fitting 63 is supported from below by a support beam 70, which is received in the interior curve defined by each U-shaped fitting. Thus, the entire weight of each cooling coil including all of its component parts (*i.e.,* upper U-turn fittings 63, vertically-oriented conduits 60 and lower U-turn fittings 62) as well as the entire contents of the cooling coil (*i.e.,* the cooling water being circulated) is supported by its respective support beam 70.

As further shown in Fig. 4, a suitable walkway or catwalk 74 is arranged between every other cooling coil at an elevation which is at or near upper U-turn fittings 63 to provide easy access to and support for any maintenance personnel that may be needed for periodic inspection and/or repair of the cooling coils.

Fig. 5 is another schematic view which illustrates how the different cooling coils in cooling coil assembly 18 are controlled. In this regard, it is common practice during operation of a conventional acrylonitrile reactor 10 to "rotate" the cooling coils, *i.e.,* periodically to shut down and then restart each cooling coil individually and serially. Most commercial ammoxidation catalysts sublime molybdenum, which normally deposits as scale on the cooling coils' exterior surfaces over time. Because this molybdenum scale adversely affects cooling coil performance, it is necessary to dislodge this molybdenum scale from time to time to keep the cooling coils functioning properly. Normally, this is done by periodically shutting down and then restarting each cooling coil, as this shut down/restart procedure induces a not-insignificant mechanical shock to the cooling coil due to the wide temperature fluctuations the cooling coil experience as a result of shutting down and then starting back up. This mechanical shock is sufficient in most instances to dislodge at least some of the molybdenum scale that may have deposited on the cooling coil surfaces and in doing so recover at least some of the heat transfer capacity of that coil. This results in stable operation over extended periods of time.

To supply cooling water to the individual cooling coils, the structure illustrated in Fig. 5 is normally used. As shown there, inlet 35 of cooling coil 61 is in fluid communication with process water inlet header 80, whose elevation is typically located below upper U-turn fittings 63. Similarly, outlet 65 of cooling coil 61 is in fluid communication with process water outlet header 62, whose elevation is typically located above upper U-turn fitting 63. Normally, process water inlet header 80 and outlet header 82 take the form of large, continuous, horizontally-oriented conduits which completely encircle reactor 10. Periodic shut down and restart of each cooling coil individually is normally accomplished through a respective shut-off valve 84 associated with the inlet 35 of that cooling coil, which in most designs is a simple on-off valve as opposed to a control valve capable of fine control of fluid flowrate.

Note, also, that shut-off valve 84 is at least one valve in cooling coil 61 between process water inlet header 80 and process water outlet header 82. That is to say, cooling coil 61 is constructed without any additional valve or other flow control device, in particular without a flow control valve associated with cooling coil outlet 65. This is because such an additional valve is unnecessary to achieve the desired operation and control of the cooling coils in the manner described above. In addition, eliminating an outlet flow control valve also eliminates the need for a safety relief valve, which would otherwise be necessary if such an outlet flow control valve were used (i.e. PSV on each individual coil would be required).

Maintaining the acrylonitrile reactor at or near its optimal reaction temperature, both throughout the reactor as a whole as well as from region to region inside the reactor, is important for good reactor performance. Moreover, good cooling coil design is also important since the rate at which heat can be withdrawn from the reactor is often the rate-limiting step which determines the maximum capacity at which the acrylonitrile reactor can operate. In addition, poor cooling coil design and/or operation can lead to excessive cooling coil corrosion, which can require premature repair, which is very expensive.

Accordingly, there is a continuing need for improvements in the design and operation of the cooling coils of a commercial acrylonitrile reactor, not only to improve reactor performance, but also to decrease conduit erosion thereby reducing down-time and repair costs.

A cooling coil assembly according to the preamble of claim 1 is disclosed in US 2009/0163756A1.

### Summary

In accordance with this invention, a series of improvements is made to the design and operation of the cooling coil assembly used in a typical oxidation or ammoxidation reactor, such as a commercial acrylonitrile reactor. As a result, not only is reactor performance improved but, in addition, the useful life of the cooling coil assembly is extended.

In an embodiment not corresponding to the invention claimed there is provided a cooling coil assembly for removing excess heat generated by an oxidation or ammoxidation reactor, the cooling coil assembly including multiple cooling coils, each cooling coil comprising multiple cooling coil courses fluidly connected to one another in series so as to define a cooling water passageway having a cooling water inlet and a cooling water outlet, each cooling coil course defining a vertically-oriented cooling coil course plane, wherein each cooling coil extends from inside the reactor towards the periphery of the reactor along a respective vertically-oriented primary cooling coil plane, and further wherein at least some of the cooling coil courses in at least one cooling coil are arranged so that their cooling coil course planes are transverse to the primary cooling coil plane of that cooling coil.
According to the invention, there is provided a cooling coil assembly for removing excess heat generated by an oxidation or ammoxidation reactor, the cooling coil assembly including single or multiple cooling coils, each cooling coil defining a cooling water flow passageway for transport of cooling water therethrough, a cooling water inlet and a cooling water outlet, each cooling coil further comprising a cooling water shutoff valve associated with its cooling water inlet, each cooling coil also being free of a valve for controlling the flow of cooling water passing through its cooling water outlet, wherein the lengths of at least some of the cooling water flow passageways are different from one another. In this aspect, a number of cooling coils is selected to provide an average percentage of cooling water converted to steam of about 15% or less.
In an embodiment not corresponding to the invention claimed, there is provided a cooling coil assembly for removing excess heat generated by an oxidation or ammoxidation reactor having walls, the cooling coil assembly including single or multiple cooling coils, each cooling coil comprising multiple cooling coil courses fluidly connected to one another in series so as to define a cooling water passageway having a cooling water inlet passing through a wall of the reactor and a cooling water outlet, wherein the cooling water inlet includes a cooling coil inlet fitting rigidly attached to the wall of the reactor and a thermal sleeve inside the cooling water inlet fitting, wherein the outside diameter of thermal sleeve is smaller than the inside diameter of cooling coil inlet fitting so as to define a thermal space therebetween.
In a last embodiment not corresponding to the claimed invention, there is provided a cooling coil assembly for removing excess heat generated by an oxidation or ammoxidation reactor, the cooling coil assembly including multiple cooling coils, each cooling coil comprising a series of cooling coil courses including a first course at the beginning of the series and a last course at the end of the series, the multiple cooling coil courses being fluidly connected to one another so as to define a cooling water passageway having a cooling water inlet and a cooling water outlet, the cooling coil assembly further comprising a cooling coil inlet header in fluid communication with the first course of each cooling coil and a cooling water outlet header in fluid communication with the last course of each cooling coil, each cooling coil further comprising a cooling water outlet conduit connecting the last course of that cooling coil with the cooling water outlet header, wherein the elevation of the cooling coil outlet header is below the elevation of the cooling coil outlet conduit of each cooling coil.

### Brief Description of the Drawings

This invention may be more readily understood by reference to the following drawings wherein:
Fig. 1 illustrates a conventional commercial acrylonitrile reactor for carrying out the ammoxidation propylene and ammonia to acrylonitrile;
Fig. 2 is a schematic view illustrating the structure and operation of a conventional cooling coil design used in the conventional commercial acrylonitrile reactor of Fig. 1;
Fig. 3 is a top view of the conventional cooling coil design of Fig. 2;
Fig. 4 is a top view similar to Fig. 3 illustrating more detail of the conventional cooling coil design of Fig. 2;
Fig. 5 is a schematic view similar to Fig. 2, but illustrating a single cooling coil 61 as well as its method of operation;
Figs. 6 and 8 are schematic illustrations of a first feature of this invention in which the cooling coils of a conventional commercial acrylonitrile reactor are more closely packaged than in a conventional design;
Fig. 7 is a top view similar to Figs. 2 and 4 illustrating only the upper U-turn fittings 63 of one cooling coil of the conventional design of these figures including the alignment of these upper U-turn fittings with one another, Fig. 7 being taken on line 7-7 of Fig. 5;
Fig. 9 is a schematic illustration of a cooling coil hanger that can be used to suspend the cooling coils of Figs. 6 and 8 from their support structure;
Fig. 10 is a schematic illustration of another feature of this invention in which is a thermal sleeve is used to protect the junction between the inlet of a cooling coil and the reactor wall through which this cooling coil inlet passes; and
Fig. 11 is a schematic illustration of still another feature of this invention in which the outlet header for receiving cooling water and steam from the cooling coils is relocated to a position which is below the tops of these cooling coils.

### DETAILED DESCRIPTION

In accordance with the first feature of this invention, a novel arrangement of the cooling coils is adopted which enables the packing of the cooling coils inside the reactor to be increased. As a result, the total surface area provided by the cooling coil assembly as a whole can be effectively increased, which in turn results in better gross control of cooling coil operation and, at least in some instances, an increase in overall reactor capacity.

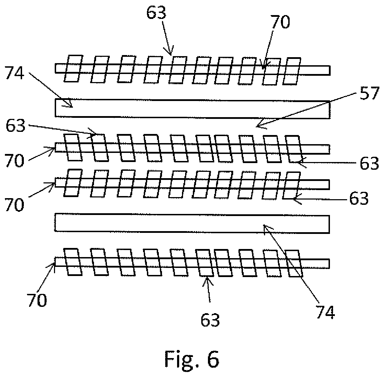

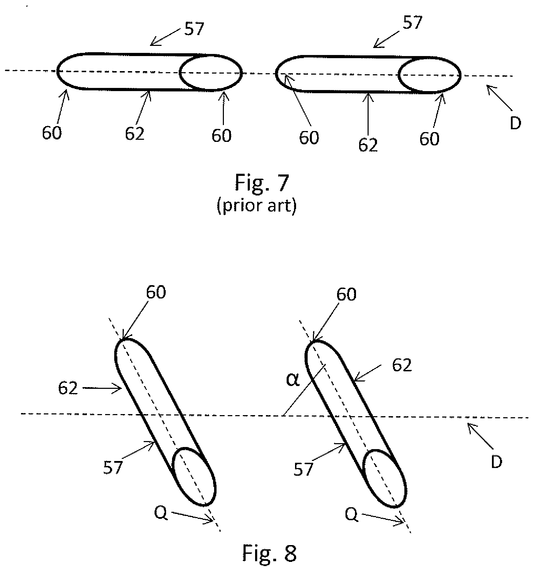

This feature is illustrated in Fig. 6, which is a schematic view similar to Fig. 4, in that it shows the arrangement of the upper U-turn fitting 63 of each cooling coil 61 and their arrangement with respect to catwalks 74 and cooling coil support beams 70 of the cooling coil assembly. *See, also,* Fig. 7, which schematically illustrates the arrangement of the coil courses in the conventional design of Figs. 2, 3, 4 and 5. Compare that with Fig. 8, which is a schematic illustration similar to Fig. 7 but illustrates the arrangement of the cooling coil courses in the inventive design of Fig. 6.

As shown in Fig. 6, upper U-turn fittings 63 of cooling coil 61 are arranged in an offset relationship with respect to one another rather than in a co-planar relationship as shown in Fig. 4. In the conventional design as illustrated in Fig. 4, cooling coil 61 extends from inside reactor 10 to the periphery of reactor 10 *(i.e.,* from location R to location S) along vertically-oriented plane D. For convenience, vertically-oriented plane D is referred to herein as the primary cooling coil plane of cooling coil 61. As further shown in Fig. 4, all of the major elements of cooling coil 61 (*i.e.,* all of the vertically-oriented cooling conduits 60 as well as all of lower U-turn fittings 62 and upper U-turn fittings 63) are co-planar, *i.e.,* they all are aligned with vertically-oriented primary cooling coil plane D in the sense that their centers or axes lie in this plane. This is further schematically illustrated in Fig. 7, which shows that cooling water conduits 60 as well as lower U-turn fittings 62 of cooling coil courses 57 are aligned with one another in the sense that their centers or axes all lie in common vertically-oriented primary cooling coil plane D. In addition, as further shown in Fig. 4, catwalks 74 are also arranged between and parallel to these major elements.

In the modified design of this aspect of the invention, however, at least some cooling coil courses 57 of at least one cooling coil are arranged transverse to the vertically-oriented primary cooling coil plane in which the cooling coil, as a whole, lies. Normally, all of the cooling coil courses 57 of at least one cooling coil are arranged in this way, while is some embodiments all of the cooling coil courses in a majority or even all of the cooling coils are arranged in this way.

This arrangement is more fully illustrated in Fig. 8, which shows that cooling water conduits 60 and lower U-shaped fittings 62 of each cooling coil course 57 of this design each lie in their own respective cooling coil course planes Q, which are arranged at an acute angle α with respect to vertically-oriented primary cooling coil plane D in which cooling coil 61, as a whole, lies. Acute angle α can be any desirable angle. In one aspect the angel is between about 30 to about 60°, and in another aspect between about 40 to about 50°.

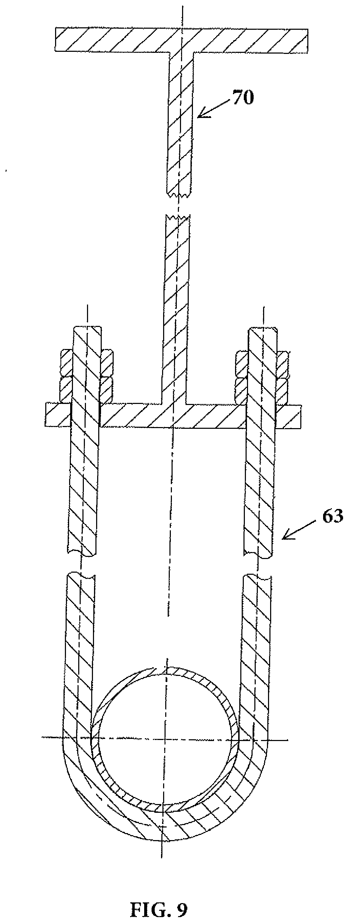

As further shown in Fig. 6, support beams 70 which carry the entire weight of cooling coils 61 and their contents are located above U-turn fittings 63 rather than below these U-turn fittings, as in the conventional design of Figs. 2, 3, 4 and 5. In addition, as shown in Fig. 9, suitable support hangers are provided for suspending each U-turn fitting 63 from its associated support beam 70.

A first advantage of the modified design of this feature of the invention is that cooling coil courses 57 can be more closely packed than in the conventional design. This enables the effective surface area of the cooling coil assembly made with this design to be increased with respect to the conventional design, which in turn achieves greater cooling capacity and has the potential for greater reactor temperature control as compared with the conventional design. The cooling coil design described herein provided more cooling coil courses per meter of reactor diameter. In this aspect, the coil design described herein is effective for providing about 40 to about 60 cooling coil courses per meter of reactor diameter, and in another aspect, about 45 to about 55 cooling coil courses per meter of reactor diameter.

A second advantage of this modified design is that the mechanical stress which is imparted to the metal elements forming each cooling coil in this design as a result of periodic shut down and restart can be better accommodated with this design compared with the conventional design. This is due to the fact that upper U-turn fittings 63 in the inventive design are suspended by hangers from, and in addition arranged transverse to, support beam 70. Accordingly, when the cooling coils of the inventive design expand and contract in response to temperature changes, less stress is imparted to these cooling coils than would otherwise be the case. This is because a significant part of this expansion and contraction occurs transversely to these support beams, and further because the hangers serve as a buffer absorbing size changes and associated movements that occur between these cooling coils and support beams.

Accordingly, as a result of this design modification, not only is it possible to increase the cooling capacity provided by a cooling coil assembly without increasing the auxiliary equipment needed to accommodate such an assembly (and in particularly the number of catwalks and support beams) but in addition it is also possible to eliminate, or at least substantially reduce, cooling coil failure and associated maintenance cost which normally arise because of mechanical stresses imparted to the cooling coils as a result of periodic shut down and restart. As indicated, the design described herein provides more coils. More coil may be cycled less frequently.

In accordance with a second feature of this invention, the cross-sectional areas of the flow passageways inside the different cooling coils of the inventive cooling coil assembly are adjusted so that the amount of cooling water which is converted into steam in each cooling coil assembly has an average of about 15% or less, in another aspect, about 10 to about 15%. Desirably, these cross-sectional areas are selected so that the amount of cooling water which is converted into steam in all of the cooling coils in this cooling coil assembly varies from one another by no more than 5%, desirably by no more than 4%, no more than 3%, no more than 2% or even no more than 1%, based on the total amount of cooling water passing through the cooling coils.

As indicated, a cooling coil assembly may include cooling coils where each cooling coil includes different numbers of cooling coil courses. For example, a cooling coil assembly may include cooling coils where the majority of cooling coils have multiple cooling coil courses (for example 6 cooling coil courses) and some cooling coils have only one cooling coil course. Removal of cooling coils effects production rates and the different numbers of cooling coil courses which can be removed in cooling coil cycling provides operational flexibility for maintaining desired production rates.

As shown especially in Fig. 2, the different cooling coils in a typical commercial acrylonitrile reactor normally do not all have the same number of cooling coil courses 57. As a result, some of these cooling coils have longer flow passageways while others have shorter flow passageways. This feature can lead to non-uniform operation of the cooling coils, because the residence time of the cooling water inside a longer flow passageway is inherently greater than the residence time of the cooling water in a shorter flow passageway. As a result, more of the cooling water in a longer flow passageway is converted to steam than in a shorter passageway. This inherently leads to higher flow velocities inside the longer flow passageways, especially near their outlet ends. This, in turn, can causes excessive erosion as well as plating out (*i.e.,* precipitation and deposition) of the minerals and other ingredients in the cooling water at these locations.

As indicated above, it is desirable in accordance with this feature of the invention that the amount of steam generated in each cooling coil assembly has an average of about 15% or less, in another aspect, about 10 to about 15%. That is to say, it is desirable that the amount of cooling water which is converted into steam in each cooling coil assembly be no more than about 15%, in another aspect, about 10 to about 15%, of the water fed to that cooling coil assembly. Therefore, in accordance with this feature of the invention, the cross sectional area of the flow passageways of each cooling coil are selected so that, when all shut-off valves 84 are in an open position, the cooling water converted to steam in each passageway will be as close as possible to one another at a value which is about 15% or less, and in another aspect, about 10 to about 15%. In this aspect, the amount of steam generated is a calculated value.

The most cost-effective way to design a commercial acrylonitrile reactor is to make each cooling coil from pipes of the same diameter and to control each cooling coil with the same shut-off valve 84, *i.e.,* each control valve is identical to the others. Therefore, the simplest way of ensuring that the cross-sectional of the area of the flow passageways of each cooling coil is selected to achieve the same conversion of water into steam is to place a suitable flow restriction inside each cooling coil, or at least inside each cooling coil with a shorter flow passageway, desirably at or near its inlet end or its outlet end or both. Determining the exact size of each flow restriction (or the relative cross-sectional areas of the flow passageways if flow restrictions are not used) can easily be done through conventional heat transfer calculations given the relative lengths of the different flow passageway and hence the different lengths of time the cooling water will be present in these different passageways.

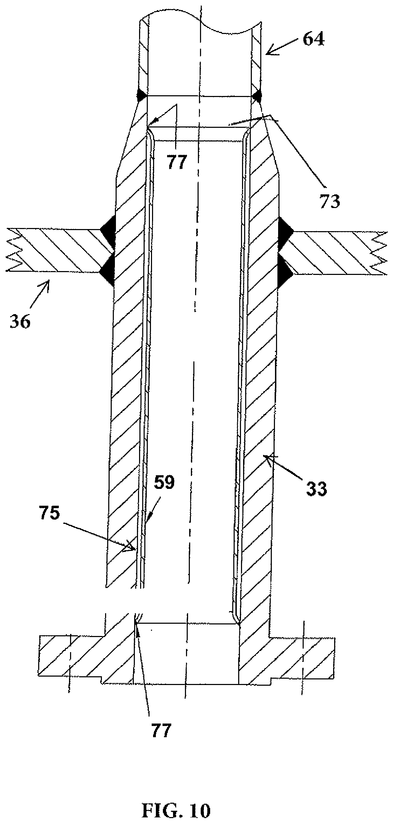

A third feature of this invention is illustrated in Fig. 10. In a conventional design such as illustrated in Figure 5, inlet line 64 of cooling coil 61 is directly welded to reactor wall 36 of reactor 10. As indicated above, it is common practice to "rotate" the cooling coils of a commercial acrylonitrile reactor by periodically shutting down and then restarting each cooling coil individually and serially. When a cooling coil is shut down, its temperature rapidly builds towards the normal operating temperature of the reactor, about 350° to about 480° C. Then, when the cooling coil is restarted by contact with additional amounts of cooling water, its temperature drops back down to at or near the boiling point of this cooling water almost instantaneously. This drop in temperature can impart substantial thermal stress to cooling coil 61, especially where its inlet line 64 is welded to reactor wall 36. Over time this repeated thermal stress can lead to mechanical failure at this location.

In accordance with this feature's invention, this problem is avoided by installing a thermal sleeve at the location where inlet line 64 of cooling coil 61 traverses reactor wall 36 of reactor 10. As shown in Fig. 10, thermal sleeve 59, which communicates with cooling coil inlet line 64, is received in cooling coil inlet fitting 33, which passes through and is welded to reactor wall 36 of reactor 10. The outside diameter of thermal sleeve 59 is slightly smaller than the inside diameter of cooling coil inlet fitting 33 so as to define a thermal space 75 therebetween, which is maintained by spacer rings 77. Outlet rim 73 of thermal sleeve 59 is not welded or otherwise permanently secured to cooling coil fitting 33 and hence is free to move, axially, with respect to this cooling coil fitting.

With this structure, any thermal stresses on the mechanical joint between cooling coil inlet line 64 and reactor wall 36 that would otherwise occur because of substantial temperature changes occurring inside cooling coil 61 when it is shut down and restarted is relieved though the expansion and contraction of thermal sleeve 59. As a result, mechanical failure of cooling coil 61 at the location where it traverses reactor wall 36 of reactor 10 is largely avoided.

In accordance with still another feature of this invention, the cooling water outlet header which is provided to receive cooling water and steam passing out of each cooling coil is relocated to a position which is below the outlet line of each cooling coil and the outlet header. In one asepct, the cooling water outlet is relocated to a position which is below the tops of the cooling coil courses of each cooling coil.

As shown in Fig. 5, in the conventional design, cooling water outlet header 82 is located above cooling water outlet line 79 as well as U-turn fittings 63, which define the tops of cooling coil courses 67, 69 and 71. As indicated above, the cooling coils of a conventional commercial acrylonitrile reactor are periodically shut down and then restarted to remove any molybdenum scale that may have deposited on their outsider surfaces. When a cooling coil is shut down, any cooling water remaining inside rapidly evaporates because the temperature inside the acrylonitrile reactor is so high. When this occurs, because there is no outlet valve associated with outlet line 79, gravity causes cooling water in outlet header 82 to flow back down into this shut down cooling coil through cooling coil outlet line 79. This results in still additional amounts of cooling water evaporating and hence being converted to steam inside the cooling coil.

Cooling water normally contains dissolved minerals as well as additional treatment chemicals. When a cooling coil is shut down, these minerals and treatment chemicals tend to precipitate out and deposit on the interior surfaces of the cooling coils, especially in lower U-turn fittings 62. The amount of these deposits can be substantial, especially if a cooling coil is shut down too long, as this allows substantial additional amounts of cooling water from cooling water outlet header 82 to flow back into and hence evaporate from this shut down cooling coil. Over time, this can cause the cross-sectional area of the flow passageway inside the cooling coil, especially at these locations, to be reduced significantly, which results in the flow rate of cooling water passing through these locations increasing substantially. This in turn can lead to significant erosion of the cooling coils at these locations and hence premature cooling coil failure.

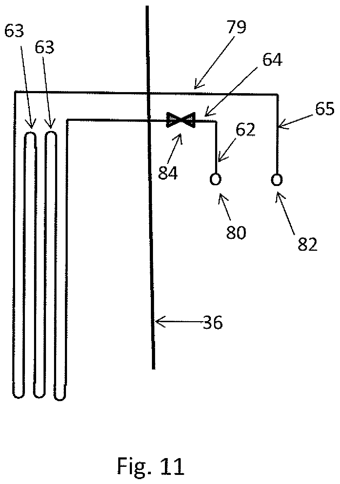

In accordance with this feature of the invention, this problem is avoided by relocating cooling water outlet header 84 to an elevation which is below outlet line 79. In one aspect, outlet header is located below the top of the last cooling coil course at least one cooling coil, more desirably below the last course of a majority or even all cooling coils. In another aspect, outlet header is located below the tops of all cooling coil courses in at least one coil, more desirably below the tops of all cooling coil courses in all cooling coils. *See,* Fig. 11, which schematically illustrates these features.

With this arrangement, flowback of additional amounts of cooling water from cooling water outlet header 32 into a shutdown cooling coil through gravity is prevented essentially completely, because cooling water outlet line 79, and in one aspect, upper U-turn fittings 63, are located too far above outlet header 82 to enable gravity to move any significant amount of cooling water back into the shutdown cooling coil.

## Claims

1. A cooling coil assembly for removing heat generated by an oxidation or ammoxidation reactor, the cooling coil assembly including multiple cooling coils, each cooling coil defining a cooling water flow passageway for transport of cooling water therethrough, a cooling water inlet and a cooling water outlet, each cooling coil further comprising a cooling water shutoff valve associated with its cooling water inlet, each cooling coil also being free of a valve for controlling the flow of cooling water passing through its cooling water outlet, **characterized in that** the lengths of at least some of the cooling water flow passageways are different from one another,
wherein the cross-sectional area of the flow passageway of each cooling coil assembly is selected so that the average percentage of cooling water converted to steam **in that** cooling coil assembly is about 15% or less.

2. The cooling coil assembly of claim 1 wherein the cross-sectional area of the flow passageway in each cooling coil assembly is selected so that the average percentage of cooling water converted to steam in that cooling coil assembly is about 10 to about 15%.

3. The cooling coil assembly of claim 1, wherein the cooling coils in the cooling coil assembly comprise at least three different lengths.

4. The cooling coil assembly of claim 1, wherein the cooling coils are made from conduits having essentially the same diameter, wherein some of the cooling coils are shorter than other of the cooling coils, and further wherein those cooling coils having shorter lengths include restrictions in their flow passageways for controlling the percentage of cooling water converted to steam.

## Patentansprüche

1. Kühlschlangenanordnung zur Abfuhr von Wärme die durch einen Oxidations- oder Ammoxidationsreaktor erzeugt wird, wobei die Kühlschlangenanordnung mehrere Kühlschlangen aufweist, wobei jede Kühlschlange einen Kühlwasser-Strömungskanal zum Transport von Kühlwasser bildet, einen Kühlwassereinlass und einen Kühlwasserauslass aufweist, wobei jede Kühlschlange ferner ein Kühlwasserabsperrventil, das mit seinem Kühlwassereinlass verbunden ist, aufweist, und wobei jede Kühlschlange ohne ein Ventil zur Steuerung des Durchflusses von Kühlwasser ist, das durch seinen Kühlwasserauslass strömt,
**dadurch gekennzeichnet, dass**
die Längen mindestens einiger der Kühlwasser-Strömungskanäle unterschiedlich sind,
wobei die Querschnittsfläche des Strömungskanals jeder Kühlschlangenanordnung so ausgewählt ist, dass der mittlere Anteil des in Dampf umgewandelten Kühlwassers in dieser Kühlwasseranordnung ungefähr 15 % oder weniger beträgt.

2. Kühlschlangenanordnung nach Anspruch 1, wobei die Querschnittsfläche des Strömungskanals in jeder Kühlschlangenanordnung so ausgewählt ist, dass der mittlere Anteil des in Dampf umgewandelten Kühlwassers in dieser Kühlschlangenanordnung ungefähr 10 bis ungefähr 15 % beträgt.

3. Kühlschlangenanordnung nach Anspruch 1, wobei die Kühlschlangen in der Kühlschlangenanordnung mindestens drei unterschiedliche Längen aufweisen.

4. Kühlschlangenanordnung nach Anspruch 1, wobei die Kühlschlangen aus Leitungen hergestellt sind, die im Wesentlichen den gleichen Durchmesser haben, wobei einige der Kühlschlangen kürzer als andere Kühlschlangen sind, wobei ferner die Kühlschlangen mit den kleineren Längen in ihren Strömungskanälen verengte Bereiche zur Steuerung des Anteils des in Dampf umgewandelten Kühlwassers enthalten.

## Revendications

1. Assemblage de serpentins de refroidissement pour éliminer de la chaleur produite par un réacteur d'oxydation ou d'ammoxydation, l'assemblage de serpentins de refroidissement incluant de multiples serpentins de refroidissement, chaque serpentin de refroidissement définissant une voie de passage d'écoulement d'eau de refroidissement pour un transport d'eau de refroidissement à travers celui-ci, une entrée d'eau de refroidissement et une sortie d'eau de refroidissement, chaque serpentin de refroidissement comprenant de plus une vanne d'arrêt d'eau de refroidissement associée à son entrée d'eau de refroidissement, chaque serpentin de refroidissement étant également exempt d'une vanne pour contrôler l'écoulement d'eau de refroidissement passant à travers sa sortie d'eau de refroidissement, **caractérisé en ce que** les longueurs d'au moins certaines des voies de passage d'écoulement d'eau de refroidissement sont différentes les unes des autres,
dans lequel la section transversale de la voie de passage d'écoulement de chaque assemblage de serpentins de refroidissement est choisie de sorte que le pourcentage moyen d'eau de refroidissement convertie en vapeur dans cet assemblage de serpentins de refroidissement est d'environ 15 % ou inférieur.

2. Assemblage de serpentins de refroidissement selon la revendication 1, dans lequel la section transversale de la voie de passage d'écoulement dans chaque assemblage de serpentins de refroidissement est choisie de sorte que le pourcentage moyen d'eau de refroidissement convertie en vapeur dans cet assemblage de serpentins de refroidissement est d'environ 10 à environ 15 %.

3. Assemblage de serpentins de refroidissement selon la revendication 1, dans lequel les serpentins de refroidissement dans l'assemblage de serpentins de refroidissement comprennent au moins trois longueurs différentes.

4. Assemblage de serpentins de refroidissement selon la revendication 1, dans lequel les serpentins de refroidissement sont constitués de conduits ayant essentiellement le même diamètre, dans lequel certains serpentins de refroidissement sont plus courts que d'autres des serpentins de refroidissement, et de plus dans lequel ces serpentins de refroidissement présentant des longueurs plus courtes comprennent des étranglements dans leurs voies de passage d'écoulement pour contrôler le pourcentage d'eau de refroidissement convertie en vapeur.
